(19) Europäisches Patentamt
European Patent Office
Office européen des brevets

(11) **EP 1 986 670 B1**

(12) # EUROPEAN PATENT SPECIFICATION

(45) Date of publication and mention
of the grant of the patent:
**28.06.2017 Bulletin 2017/26**

(51) Int Cl.:
*A61K 36/185* (2006.01)    *A61K 31/352* (2006.01)
*A61P 3/04* (2006.01)    *A61P 3/10* (2006.01)
*A61K 31/70* (2006.01)

(21) Application number: **07733897.8**

(22) Date of filing: **20.02.2007**

(86) International application number:
**PCT/IB2007/000400**

(87) International publication number:
**WO 2007/096739 (30.08.2007 Gazette 2007/35)**

(54) **INTESTINAL ALPHA-GLUCOSIDASE INHIBITORS AND A PROCESS FOR THE ISOLATION AND USE THEREOF**

INTESTINALE ALPHA-GLUCOSIDASE-INHIBITOREN UND VERFAHREN ZUR ISOLIERUNG UND VERWENDUNG DAVON

INHIBITEURS DE L'ALPHA-GLUCOSIDASE INTESTINALE ET PROCÉDÉ POUR ISOLER ET UTILISER CEUX-CI

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.02.2006 IN DE04772006**

(43) Date of publication of application:
**05.11.2008 Bulletin 2008/45**

(73) Proprietor: **Council of Scientific and Industrial Research**
**New Delhi 110 001 (IN)**

(72) Inventors:
• **RAO, Janaswamy, Madhusudana**
**Andhra Pradesh (IN)**
• **KATRAGADDA, Suresh, Babu**
**Andhra Pradesh (IN)**
• **TIWARI, Ashok, Kumar**
**Andhra Pradesh (IN)**
• **TATIPAKA, Hari, Babu**
**Andhra Pradesh (IN)**
• **PULLELA, Venkata, Srinivas**
**Andhra Pradesh (IN)**
• **SURYADEVARA, Praveen, Kumar**
**Andhra Pradesh (IN)**
• **SASTRY, Boggavarapu, Subrahmanya**
**Andhra Pradesh (IN)**
• **ALI, Amtul, Zehra**
**Andhra Pradesh (IN)**

• **YADAV, Jhillu, Singh**
**Andhra Pradesh (IN)**

(74) Representative: **Nederlandsch Octrooibureau**
**P.O. Box 29720**
**2502 LS The Hague (NL)**

(56) References cited:
**WO-A-2004/045605    WO-A-2007/080484**

• **DATABASE WPI Week 199539 Derwent Publications Ltd., London, GB; AN 1995-299494 XP002477794 & JP 07 196490 A (TERUMO CORP) 1 August 1995 (1995-08-01)**
• **SANKARA SUBRAMANIAN S ET AL: "FLAVONOIDS OF THE STEM BARK OF OROXYLUM-INDICUM" CURRENT SCIENCE (BANGALORE), vol. 41, no. 2, 1972, pages 62-63, XP009099109 ISSN: 0011-3891**
• **NISHIOKA T ET AL: "Baicalein, an alpha-glucosidase inhibitor from Scutellaria baicalensis" JOURNAL OF NATURAL PRODUCTS, XX, XX, vol. 61, no. 11, 1998, pages 1413-1415, XP003011527 ISSN: 0163-3864**
• **GAO HONG ET AL: "Structure-activity relationships for alpha-glucosidase inhibition of baicalein, 5,6,7-trihydroxyflavone: the effect of A-ring substitution." BIOSCIENCE, BIOTECHNOLOGY, AND BIOCHEMISTRY FEB 2004, vol. 68, no. 2, February 2004 (2004-02), pages 369-375, XP009099477 ISSN: 0916-8451**

**(Cont. next page)**

- DATABASE WPI Week 200645 Derwent Publications Ltd., London, GB; AN 2006-439739 XP002477795 & KR 2005 037 102 A (KOREA INST ORIENTAL MEDICINE) 21 April 2005 (2005-04-21)
- MATSUI TOSHIRO ET AL: "Luteolin, a flavone, does not suppress postprandial glucose absorption through an inhibition of alpha-glucosidase action" BIOSCIENCE BIOTECHNOLOGY AND BIOCHEMISTRY, vol. 66, no. 3, March 2002 (2002-03), pages 689-692, XP002477793 ISSN: 0916-8451

- DATABASE WPI Week 200151 Derwent Publications Ltd., London, GB; AN 2001-473598 XP002477796 & KR 2001 009 475 A (KIM B G) 5 February 2001 (2001-02-05)

## Description

### Field of the invention

This invention relates to α-glucosidase inhibitors isolated from *Oroxylum indicum* an Indian medicinal plant. Particularly, the invention relates to hexane and acetone fraction of *Oroxylum indicum* wherein the hexane fraction comprises α-glucosidase active compounds of oroxylin A, chrysin and baicalein and acetone fraction comprises the compounds of oroxylin A, chrysin, baicalein, methoxy chrysin and oroxyloside methyl ester. This invention also relates to a process for the isolation of α-glucosidase inhibitory active compounds from the above said hexane and acetone fraction of *Oroxylum indicum.*

This invention also relates to the use of these potent intestinal α- glucosidase inhibitors as active ingredients that can be used in pharmaceuticals, food products, health foods and specialized health care foods as anti hyperglycemic agent for prevention and treatment of post prandial hyperglycemia, hyperglycemia and related conditions in diabetes mellitus, obesity and disease conditions requiring hyperglycemic control as well as inhibition of α-glucosidase activity.

### Background of the invention

The frequency of diabetes, obesity and hyperlipidemia in the population worldwide is high and continue to increase. The global economic prosperity along with adoption of Western habits and lifestyle are dragging these diseases toward global epidemic. Diabetes mellitus is one of the most serious chronic disease that is developing with an increase in obesity and ageing in the general population. Though there are drugs developed for treatment of diabetic. syndrome, still today the most challenging goals in the management of patients with diabetes mellitus is to achieve blood glucose levels as close to normal as possible. In addition, the post-prandial hyperglycemia or hyperinsulinemia are independent risk factors for the development of macrovascular complications of diabetes mellitus. A reasonable approach to control this carbohydrate metabolism dependent disease particularly, the post-prandial hyperglycemia is to slow down digestion of carbohydrates and hence retard the absorption of glucose released due to the digestion of carbohydrates. The most feasible and reasonable way to achieve this task is to limit the activity of carbohydrate hydrolyzing enzymes, more specifically, the intestinal α-glucosidase. Intestinal α-glucosidase plays an important role in carbohydrate digestion and hence, absorption of glucose. Thereby inhibition of intestinal α-glucosidase offers potential and effective opportunity to control the post-prandial hyperglycemic burden. Drugs like acarbose, miglitol and voglibose are known to reduce post-prandial hyperglycemia primarily by interfering with carbohydrate digesting enzyme α-glucosidase and thereby delaying glucose absorption (Moorardian AD and Thurman JE, Drugs 57, 19-29, 1999) and are currently being used clinically as medicines for diabetic and obese patients. Furthermore, intestinal α-glucosidase inhibitors are also being advocated for weight control, prevention of weight gain, weight loss and treatment and prevention of obesity and related disorders. (Rosner Harvey, US patent 2004122054).

Though above mentioned α-glucosidase inhibitors are in clinical practice, their application finds limitations due to their side effects like abdominal pain, flatulence, diarrhea etc in the colon (Uchida, R, Nasu, A et. al Chem.. Pharm. Bull 47, 187-193, 1999). Due to these limitations therefore, there is felt an urgent need to look for therapy from natural sources particularly phytochemicals from medicinal plants and build up of their pool because increasing lot of the patients can develop resistance to current regimens in future.

The screening of natural sources has led mankind to the discovery of many clinically useful drugs that play major role not only in the treatment but also prevention of diseases discussed above. Therefore, increasing clinical importance of epidemics of diabetes, obesity and related cardiovascular disorders as well as drug resistance has put additional burden on scientific community and therefore urgency for identifying novel natural resources bearing substantial amounts of active compounds and their development as medicament. In the light of above descriptions, our search for intestinal α-glucosidase inhibitors for the control of post-prandial hyperglycemia, hyperglycemic conditions in diabetes mellitus as well as prevention of weight gain, facilitate weight loss, and control of weight in prevention and treatment of obesity and related disorders, led to the identification of *Oroxylum indicum,* which contained in substantial yields potent mammalian intestinal α-glucosidase inhibitors for the first time.

*Oroxylum indicum* Vent has been advocated in traditional medical practice of India for several diseases. In folklore medicine in India, the powdered stem bark is used to treat dysentery, diarrhea, sore throat, and cough and bone fractures (Kausik, P and Dhaman A. K, The medicinal plants and crude drugs of India, 2000, 398).

Present invention relates to the identification of mixtures containing potent intestinal α-glucosidase inhibitors and isolation of potent intestinal α-glucosidase inhibitor molecules from active extracts of *Oroxylum indicum,* which may find preventive as well as therapeutic applications for the control of post prandial hyperglycemia, hyperglycemic conditions in diabetic syndrome, weight control and weight loss along with other complications in obesity. We identify *Oroxylum indicum* an Indian medicinal plant to possess potent intestinal α-glucosidase inhibitors. The hexane extract of dried stem bark of *Oroxylum indicum* constitutes 95% of three major active principles identified as Oroxylin A, Chrysin, Baicalein and acetone extract contains five major active principles, that contains apart from Oroxylin A, Chrysin, Baicalein, two more compounds namely a new intestinal $\alpha$-glucosidase inhibitors oroxyloside methyl ester and methoxy chrysin in substantial yields. These mixtures and molecules may find preventive as well as therapeutic application in controlling post-prandial hyperglycemia, hyperglycemic conditions in diabetes mellitus, obesity along with disorders and diseases that require inhibition of $\alpha$-glucosidase enzyme.

Applications, Administration and the formulations:-

These intestinal $\alpha$-glucosidase inhibitors may be applied or administrated by any suitable conventional method prevalent in pharmaceutical practice for the control of post-prandial hyperglycemia, treatment of hyperglycemic conditions in diabetes mellitus, control and reduction weight and other risk factors in obesity, and also in disease condition requiring inhibition of $\alpha$-glucosidase. For human, animals and or veterinary application as the case may be intestinal $\alpha$-glucosidase inhibitory mixtures or molecules may preferably be taken orally approximately half an hour before or prior to the respective meals or the snacks in order to inhibit the action of $\alpha$-glucosidase enzyme on its substrate carbohydrate and potentiate the mechanism of action and hence impart better therapeutic action.

The intestinal $\alpha$-glucosidase inhibitory mixtures or the compounds present in mixtures of this disclosure may be formulated with any of the suitable pharmaceutically acceptable additive, carrier, vehicle, food preparations etc suitable for human application. The materials should be selected such that they should not interfere with the potency and the property of the mixture or the molecule but materials that can add to or improve the activity, may be preferred and may be decided by the conventional art and the skills available in formulary.

Effective Dose:-

Effective dose level and duration of drug administration may be decided by the skill of ordinary art in order to bring therapeutic parameter of the disease under consideration under the control. The actual rate, amount of applications, and the time of administration may vary depending upon the disease condition and severity and may be irrespective of the concentration and duration as described in the examples.

## Objects of the invention

The main object of the invention is to provide an active extracts possessing potent intestinal $\alpha$-glucosidase inhibitory property.

Another objective of the invention is to provide active principles from potential extract namely hexane extract and acetone extract as intestinal $\alpha$-glucosidase inhibitors.

Yet another object of the invention is to provide a process for the isolation of active principles from active hexane/acetone extract of *Oroxylum indicum* as Oroxylin A, Baicalein, Chrysin, Methoxycrysin and Oroylosidemethyl ester as intestinal $\alpha$-glucosidase inhibitors.

Yet another object of the invention is to assign new activity as intestinal $\alpha$-glucosidase inhibitor to compounds Oroxylin A, Chrysin, Methoxychrysin and Oroxylosidemethyl ester isolated from either acetone extract or the hexane extract of plant *Oroxylum indicum* Yet another object of the invention is to provide a therapeutic application of the extracts of hexane and /or acetone of *Oroxylum indicum* and compounds isolated from these extracts as intestinal $\alpha$-glucosidase inhibitor for control of post-prandial hyperglycemia, elevated glucose level in diabetes mellitus and weight control in obesity. Still another object of the present invention is to provide active extracts and molecules from *Oroxylum indicum* for therapeutic use as $\alpha$-glucosidase inhibitor for prevention and treatment of diseases involving inhibition of $\alpha$- glucosidase enzyme.

## Summary of the invention

The present invention is defined by the claims. The present invention provides $\alpha$ glucosidase inhibitory active fraction of plant *Oroxylum indicum*, selected from hexane and acetone extract of plant oroxylum indicum, wherein said hexane extract comprises oroxylin A, chrysin and baicalain and wherein said acetone extract comprises oroxylin A, chrysin, baicalain, methoxy chrysin and oroxyloside methyl ester, for use in in the treatment of post prandial hyperglycemia, hyperglycemia, diabetes mellitus, obesity and disease conditions requiring hyperglycemic control.

In yet another embodiment the hexane extract of plant *Oroxylum indicum* comprises active compounds of oroxylin A, chrysin and baicalein and unidentified compounds in a ratio of about 60:10:25:5.0 (wt%).

In yet another embodiment the acetone extract comprises the active compounds of oroxylin A, chrysin, baicalein, methoxy chrysin, oroxyloside methyl ester and unidentified compounds in a ratio of about 6.7:8.3:50:16.7:13.3:5.0 (wt%).

In yet another embodiment the in vitro $\alpha$-glucosidase inhibitory activity $IC_{50}$ of the hexane/ acetone extract of *Oroxylum indicum* is in the range of 84 to 125μg/mL

In yet another embodiment the hexane fraction of plant *Oroxylum indicum* exhibits in vitro α-glucosidase inhibitory activity IC$_{50}$ of about 85 μg/mL.

In yet another embodiment the acetone extract of plant *Oroxylum indicum* exhibits in vitro α-glucosidase inhibitory activity IC$_{50}$ of about 124 μg/mL.

The isolation of α glucosidase inhibitory active compounds from hexane and/or acetone extract of plant *Oroxylum indicum,* may comprise defatting the dried stem bark of *Oroxylum indicum* with petrol, followed by extraction with hexane and/or acetone and filtering the resultant hexane or acetone extract to obtain the residue and successively eluting the resultant residue in a chromatographic column with 1-5% methanol in chloroform to obtain the desired α glucosidase inhibitory active compounds of oroxylin A, chrysin, baicalein, methoxy chrysin and oroxyloside methyl ester.

The isolation of α glucosidase inhibitory active compounds from hexane extract of plant *Oroxylum indicum,* may comprise the steps of:

a) defatting the dried stem bark of *Oroxylum indicum* with petrol, followed by extraction with hexane, concentrating and filtering the resultant hexane extract to obtain the obtain the residue,
b) subjecting the residue obtained in step(a) to elution in a chromatographic column with about 1 % methanol in chloroform to obtain the oroxylum A and residue,
c) subjecting the residue obtained in step (b) to elution with 2% methanol in chloroform to obtain the chrysin and
d) further subjecting the remaining residue to elution with 3% methanol in chloroform to obtain the baicalein.

The isolation of α glucosidase inhibitory active compounds from acetone extract of plant *Oroxylum indicum,* may comprise the steps of:

a) defatting the dried stem bark of *Oroxylum indicum* with petrol,<'> followed by successive extraction with hexane and acetone, respectively and evaporating the resultant acetone extract to obtain the dark brown coloured residue,
b) subjecting the residue obtained in step (a) to elution in a chromatographic column with about 1 % methanol in chloroform to obtain the oroxylum A and residue,
c) subjecting the residue obtained in step (b) to elution with 2% methanol in chloroform to obtain the chrysin and residue
d) subjecting the residue obtained in step (c) to elution with 3% methanol in chloroform to obtain the baicalein
e) subjecting the residue obtained in step (d) to elution with 4% methanol in chloroform to obtain the methoxy chrysin and
f) subjecting the remaining residue to elution with 5% methanol in chloroform to obtain the oroxyloside methyl ester.

The yield of Oroxylin A obtained is about 60% with respect to dried stem bark with hexane extract. The yield of chrysin obtained is about 10% with respect to dried stem bark with hexane extract. The yield of methoxy chrysin obtained is about 13.3% with respect to dried stem bark with hexane extract. The yield of oroxyloside methyl ester obtained is about 16.7% with respect to dried stem bark with hexane extract. The purity of α-glucosidase inhibitory active compounds obtained is about 95%. The in vitro α-glucosidase inhibitory activity IC$_{50}$ of oroxylin-A is 25.90μg/mL The in vitro α-glucosidase inhibitory activity IC$_{50}$ of chrysin is 57.59 μg/mL. The in vitro α-glucosidase inhibitory activity IC$_{50}$ of baicalein is 38.71 μg/mL. The in vitro α-glucosidase inhibitory activity IC$_{50}$ ofmethoxy chrysin is 95.39.71 μg/mL. The in vitro α-glucosidase inhibitory activity IC$_{50}$ of oroxyloside methyl ester is 97.31 μg/mL.

The use of alpha glucosidase inhibitory compounds in a subject typically comprises administering the hexane and/or acetone extract, optionally along with pharmaceutically acceptable carrier, addjuvants and/or diluents.

The present disclosure thus relates to a new activity for Oroxylin A and Chrysin isolated from *Oroxylum indicum* as intestinal α-glucosidase inhibitor for the first time.

**Brief Description of the Drawings**

The invention is illustrated by the accompanying drawings where in

FIG:1 represents formula of Oroxylin-A [5,7-Dihydroxy-6-methoxy-2-phenyl-chromen-4-one]
FIG:2 represents formula of Chrysin [5,7-dihydroxy-2-phenyl-chromen-4-one]
FIG:3 represents formula of Baicalein [5,6,7-trihydroxy-2-phenyl-chromen-4-one]
FIG:4 represents formula of Methoxy chrysin [5-hydroxy-7-methoxy-2-phenyl-chromen-4-one]
FIG:5 represents formula of Oroxoloside methyl ester [3,4,5-trihydroxy- 6-(6-methoxy- 4-oxo-2-phenyl-4-H-chromen-7-yoloxy) tetrahydro- pyran-2-carboxylic acid methyl ester]

**Detail description of the invention**

A new source namely *Oroxylum indicum* dried stem bark possessing substantial amount of intestinal $\alpha$-glucosidase inhibitory active principles has been identified.

Acetone extract as well as hexane extract of stem of *Oroxylum indicum* possess rich content of intestinal $\alpha$-glucosidase inhibitors.

Invention further identifies the use of above extracts as intestinal $\alpha$-glucosidase inhibitor that can be applied and or administered for the control of post-prandial hyperglycemia, hyperglycemia in diabetes mellitus and related disease conditions.

This invention further identifies use of above intestinal $\alpha$-glucosidase inhibitor to control the increase and facilitate the decrease of weight in obesity. This Invention identifies a mixture in substantial yields (1.0 %) from hexane extract of *Oroxylum indicum* and identifies that this potent intestinal $\alpha$-glucosidase inhibitory mixture constitutes about 95% of active principles isolated as Oroxylin-A (60% yield), Chrysin (10% yield) and Baicalein (25% yield). Further more this invention also identifies an other extract namely, acetone extract (1.5% yield) possessing potent intestinal $\alpha$-glucosidase inhibitors, containing along with Oroxylin-A (6.7% yield), Chrysin (8.3% yield), Baicalein(50% yield) and a new naturally occurring intestinal $\alpha$-glucosidase inhibitor as oroxyloside methyl ester (13.3% yield) and another intestinal $\alpha$-glucosidase inhibitor identified as 7-methoxy chrysin in 16.7% yield, isolated for the first time from *Oroxylum indicum.*

The disclosure identifies use of these $\alpha$-glucosidase inhibitors in disease conditions where inhibition of this enzyme play important role. Further more, it identified oroxylin-A, chrysin and baicalein as the principal active molecules in hexane extract possessing intestinal $\alpha$-glucosidase inhibitory property. The present disclosure further identifies Oroxylin-A as superior intestinal $\alpha$-glucosidase inhibitor than Baicalein, Chrysin and Oroxylin-A as the major active principle in hexane extract as intestinal $\alpha$-glucosidase inhibitor.

In accordance with the objectives of this invention the present invention again identifies acetone extract of the dried stem bark of *Oroxylum indicum* presenting as intestinal $\alpha$-glucosidase inhibitory activity.

The invention identifies the presence of oroxylin-A, baicalein, chrysin, methoxy chrysin and oroxylosidemethyl ester as principle active molecules presenting intestinal $\alpha$-glucosidase inhibitory property in acetone extract.

The present invention also identifies for the first time oroxyloside methyl ester as new naturally occurring compound from acetone extract of *Oroxylum indicum.*

In a feature of the present disclosure compound methoxy chrysin is isolated for the first time from acetone extract of *Oroxylum indicum.*

In another feature of the present disclosure oroxyloside methyl ester possesses intestinal $\alpha$-glucosidase inhibitory potential.

In yet another feature of the present disclosure compound methoxy chrysin posses intestinal $\alpha$-glucosidase inhibitory property.

In yet another feature of the disclosure, Oroxylin-A, chrysin and Baicalein also posses intestinal $\alpha$-glucosidase inhibitory property.

The present disclosure provides process for the isolation of Oroxylin-A, Chrysin, Baicalein, Methoxy chrysin and Oroxyloside methyl ester as intestinal $\alpha$-glucosidase inhibitors from *Oroxylum indicum,* the said process comprises following steps of (1).

    a) extraction of dried stem bark of *Oroxylum indicum* with Hexane
    b) extract was filtered to afford solid separate out
    c) subjecting the residue to a first elution with 1% methanol in chloroform to obtain Oroxylum-A and
    d) subjecting the residue (step c) to a second elution with 2% methanol in chloroform to obtain Chrysin and
    e) subjecting the residue (step d) to a third elution with 3% methanol in chloroform to obtain Baicalein

In a further aspect, the present disclosure relates to the isolation of these three compounds namely Oroxylin-A, Chrysin and Baicalein from *Oroxylum indicum* with hexane extract.

Further more all these compounds isolated from *Oroxylum indicum* shows the intestinal $\alpha$-glucosidase inhibition for the first time.

Further more extraction of dried stem bark of *Oroxylum indicum* with acetone, and process for isolation of compounds along with Oroxylin-A, Chrysin and Baicalein, compounds Methoxy chrysin and Oroxyloside methyl ester the said process comparing steps of

    a) subjecting the extract to obtain the residue
    b) subjecting the residue to a first elution with 1% methanol in chloroform to obtain Oroxylum-A and
    c) subjecting the residue (step b) to a second elution with 2% methanol in chloroform to obtain Chrysin and
    d) subjecting the residue (step c) to a third elution with 3% methanol in chloroform to obtain Baicalein

e)subjecting the residue (step d) to fourth elution with 4 % methanol in chloroform to obtain Methoxy chrysin

f) subjecting the residue (step e) to a fifth elution with 5%methanol in chloroform to obtain Oroxyloside methyl ester

In a further aspect, the present disclosure relates to the isolation of these five compounds namely Oroxylin-A, Chrysin, Baicalein, Methoxy chrysin and Oroxyloside methyl ester from acetone extract showing intestinal $\alpha$-glucosidase inhibition for the first time. Further disclosure relates to the isolation of compounds Methoxy chrysin and Oroxyloside methyl ester form *Oroxylum indicum* an entirely new source. In a further aspect, the present disclosure relates to isolation of fifth compound namely Oroxyloside methyl ester which is 7-O-$\beta$-D Glucuronopyranoside derivative from *Oroxylum indicum* as an entirely new compound.

Further more all these compounds from *Oroxylum indicum* shows the intestinal $\alpha$-glucosidase inhibition for the first time.

Present invention and disclosure also provide a new activity assigned to above extracts and compounds for use in control of post-prandial hyperglycemia, hyperglycemic conditions in diabetes mellitus and prevention and treatment of cause and course of diabetes and obesity related to the therapeutic activity of $\alpha$-glucosidase inhibition.

Present disclosure further opens avenues for the use as above $\alpha$-glucosidase inhibitors in disease conditions where inhibition of $\alpha$-glucosidase enzyme imparts beneficial effects for prevention and treatment of diseases involving inhibition of $\alpha$-glucosidase enzyme.

Present disclosure also provides isolation of Baicalein, methoxy chrysin and oroxyloside methyl ester from a new source namely *Oroxylum indicum* in substantial yields for the first time.

Oroxylum-A obtained from *Oroxylum indicum* has the following spectrochemical and physical properties MP:231-232°C. IR (KBr)$\nu_{max}$ 3435, 2825, 1622, 1016 cm$^{-1}$. $^1$H NMR (200 MHz, CDCl$_3$+ MeOH-d$_4$) ($\delta$) 7.82-7.86 (2H, m, H-2', 6'), 7.42-7.56 (3H, m, H-3', 4', 5'), 6.62 (1 H, s, H-8), 6.58 (1 H, s, H-3), 3.96 (3H, s, Ar-OMe). $^{13}$C NMR (50 MHz, DMSO d$_6$) $\delta$ 163.37 (C-2), 104.46 (C-3), 182.31 (C-4), 152.64 (C-5),130.80 (C-6), 157.62 (C-7), 94.49 (C-8), 152.79 (C-9), 104.71 (C-10), 131.60 (C-1'), 126.42 (C-2'), 129.20 (C-3'), 132.06 (C-4'), 60.06 (OMe). EIMS:284 (M$^+$, 100).

Chrysin obtained from *Oroxylum indicum* has the following spectralchemical and physical properties MP:285-286°C. IR (KBr)$\nu_{max}$ 3450, 2925, 1626, 1024 cm$^{-1}$. $^1$H NMR (400 MHz, CDCl$_3$+ MeOH-d$_4$) ($\delta$) 7.82-7.92 (2H, m, H-2', 6'), 7.44-7.58 (3H, m, H-3', 4', 5'), 6.64 (1 H, s, H-8), 6.44 (1 H, s, H-3), 6.24 (1 H, s, H-6). $^{13}$C NMR (50 MHz, DMSO d$_6$) $\delta$163.0 (C-2), 105.0 (C-3), 181.6 (C-4), 161.5 (C-5), 99.0 (C-6), 164.3 (C-7), 94.0 (C-8), 157.3 (C-9), 104.0 (C-10), 138.7 (C-1'), 126.1 (C-2'), 128.8 (C-3'), 131.6 (C-4'), 128.8 (C-5'), 126.1 (C-6'). EIMS: M$^+$ 254.

Baicalein obtained from *Oroxylum indicum* has the following spectralchemical and physical properties MP:223-226°C. IR(KBr)$\nu_{max}$ 3450,2920,1622,1024 cm$^{-1}$. $^1$H NMR (400 MHz, CDCl$_3$+ CDCl$_3$) ($\delta$)7.82-7.98 (2H, m, H-2', 6'), 7.44-7.60 (3H, m, H-3', 4', 5'), 6.62 (1 H, s, H-8), 6.58 (1 H, s, H-3). $^{13}$C NMR (50 MHz, DMSO d$_6$) $\delta$162.9 (C-2), 104.5 (C-3), 182.1 (C-4), 147.0 (C-5), 129.3 (C-6), 153.7 (C-7), 94.0 (C-8), 149.9 (C-9), 104.3 (C-10), 131.0 (C-1'), 126.2 (C-2'), 129.0 (C-3'), 131.7 (C-4'), 129.0 (C-5'),126.2 (C-6) EIMS:270 (M$^+$, 100).

Methoxy chrysin obtained from *Oroxylum indicum* has the following spectralchemical and physical properties MP:164°C. (KBr) $\nu_{max}$ 3450, 2925, 1654, 1621, 1016 cm$^{-1}$. $^1$H NMR (200 MHz, CDCl$_3$) ($\delta$)13.0(1H, s, OH-5), 7.82-7.96 (2H, m, H-2', 6'), 7.44-7.60 (3H, m, H-3', 4', 5'), 6.62 (1H, s, H-8), 6.60 (1 H, s, H-3), 6.58 (1 H, s, H-6), 3.96 (3H, s, OMe). $^{13}$C NMR (300 MHz, CDCl$_3$) ($\delta$)164.08 (C-2), 105.04 (C-3), 182.88 (C-4), 164.08 (C-5), 93.97 (C-6), 153.31 (C-7), 93.97 (C-8), 153.31(C-9), 105.50 (C-10), 130.96 (C-1'), 126.24 (C-2'), 128.88 (C-3'), 131.26 (C-4'), 128.88 (C-5'), 126.24 (C-6'), 60.63 (Ar-OMe).EIMS:192 (M$^+$, 100).

Oroxyloside methyl ester obtained from *Oroxylum indicum* has the following spectralchemical and physical properties

MP:201°C. UV $\lambda_{max}$ (MeOH)345, 285 nm. IR (KBr) $\nu_{max}$ 3395, 2924, 1735 (ester-C=O), 1618 (-C=O), 1461, 1359, 1224, 1076 cm$^{-1}$. $^1$H NMR (200 MHz, DMSO-d$_6$) ($\delta$)

12.78 (1H, s, OH-5), 7.90-8.0 (2H, m, H-2', 6'), 7.48-7.60 (3H, m, H-3', 4', 5'), 6.84 (1H, s, H-8), 6.80(1H, s, H-3), 3.4-5.50 (m, sugar protons), 3.78 (3H, s, OMe), 3.82 (3H, s, Ar-OMe).$^{13}$C NMR (300 MHz, DMSO-d$_6$) ($\delta$) 163.72 (C-2), 104.95 (C-3), 182.37 (C-4), 152.52 (C-5), 132.04 (C-6), 156.08 (C-7), 94.07 (C-8), 152.17 (C-9), 106.12 (C-10), 130.59 (C-1'), 126.35 (C-2',6'), 129.03 (C-3',5'), 132.06 (C-4'), 99.49 (C-1"),75.60 (C-2"), 75.25 (C-3"), 72.77 (C-4"), 71.18 (C-5"), 168.96 (C-6"), 60.21 (Ar-OMe), 51.81 (OMe). EIMS: 475 (M$^+$+1, 100).

The following examples are given by the way of illustration and therefore should not be construed to limit the scope of the invention.

**Example 1**

**Experimental protocol: process of isolation of Oroxylin-A, Chrysin and Baicalein.**

The dried powdered stem bark (200g) was first defatted with petrol in a soxhlet apparatus. The bright yellow colored powdered solid was obtained after the filtration of the hexane extract. The solid (2g) was chromatographed over silica gel (60-120 mesh), 3.5cm dia column loaded to a height of 60cm.

The column was successively eluted with 1%methanol in chloroform to afford Oroxylin-A.

The yield of Oroxylin-A is around 1.2g

Further elution of the column with 2%methanol in chloroform afforded chrysin The yield of Chrysin is around 0.2g

Further elution of the column with 3%methanol in chloroform afforded Baicalein The yield of Baicalein is around 0.5g

The ratio of the three active compounds along with unidentified compounds in hexane fraction such as Oroxylin-A, Chrysin, Baicalein and unidentified compounds are in the following ratio 60:10:25:5.0.

**A process of isolation of Methoxy chrysin and Oroxyloside methyl ester:**

The dried powdered stem bark (200g) was successively extracted with hexane and acetone. The acetone extract on evaporation afforded a dark brown colored residue (3g). The residue was chromatographed over silica gel (60-120mesh), 3.5cm dia column loaded to a height 60cm. In addition to oroxylin-A, Chrysin and Baicalein two more compounds namely Methoxychrysin and Oroxyloside methyl ester were isolated as follows.

The column was successively eluted with 1%methanol in chloroform to afford Oroxylin-A.

The yield of Oroxylin-A is around 0.2g

Further elution of the column with 2%methanol in chloroform afforded chrysin The yield of Chrysin is around 0.25g

Further elution of the column with 3%methanol in chloroform afforded

The yield of Baicalein is around 1.5g

Further elution of the elution successively with 4%methonal in chloroform afford Methoxy chrysin.

The yield of methoxy chrysin is around 0.5g

Further elution of column with 5% methanol in chloroform afford Oroxyloside methyl ester. The yield of Oroxyloside methyl ester is around 0.4g.

All the above compounds were isolated in 95% purity.

The ratio of the five active compounds along with unidentified compounds in acetone fraction such as Oroxylin-A, Chrysin, Baicalein, Methoxy chrysin, Oroxyloside methyl ester and unidentified compounds are in the following ratio 6.7:8.3:50:16.7:13.3:5.0. f

The petrochemical and physical properties of the all the above compounds are discussed earlier.

**Example 2: Assay for intestinal $\alpha$-glucosidase inhibition:-**

Rat intestinal acetone powder (sigma chemicals, USA) in normal saline in ration of 100:1 (w/v) was sonicated properly. Supernatant obtained after centrifugation at 3000rpm x 30 min at 25°C was treated as crude source of mammalian intestinal $\alpha$-glucosidase 10 $\mu$L of test samples prepared from either extracts or the molecules (5mg/ml in DMSO) were incubated in 100$\mu$L of 100 mM phosphate buffer (pH6.8) with 50 $\mu$L of crude enzyme for 5 minutes. Thereafter 50 $\mu$L of substrate (5mM, *p*-nitrophenyl $\alpha$-D-glucopyranoside prepared in the buffer)was added. After 5 minutes incubation, released p-nitro phenol was read at 405nm spectrophotometrically. For control and blank readings at the place of test samples 10 $\mu$L of DMSO and at the place of substrate 50 $\mu$L of buffer was added: In order to compensate interference from colored compounds there individual blanks were prepared and spectophotpmetric reading were evaluated accordingly the percent inhibition was calculated as follows:-

$$\left(1- \frac{\text{absorbance of the test sample}}{\text{absorbance of the control sample}}\right) \quad \text{x100}$$

and 50% inhibitory concentration($IC_{50}$)for test samples were calculated by applying suitable regression analysis from the data obtain from serial dilution of the test samples concentrations. Each test sample and other control samples were run in triplicate.

**Table 1**

| Intestinal $\alpha$-glucosidase inhibitory activity of compounds | | |
| --- | --- | --- |
| S.NO. | COMPOUND NAME | $IC_{50}$ VALUES ($\mu$g/mL) |
| 1 | Oroxylin-A | 25.90 |
| 2 | Chrysin | 57.59 |

(continued)

| Intestinal α-glucosidase inhibitory activity of compounds | | |
|---|---|---|
| S.NO. | COMPOUND NAME | IC$_{50}$ VALUES (μg/mL) |
| 3 | Baicalein | 38.71 |
| 4 | Methoxy chrysin | 95.39 |
| 5 | Oroxyloside methyl ester | 97.31 |
| 6 | Hexane extract | 84.16 |
| 7 | Acetone extract | 124.03 |

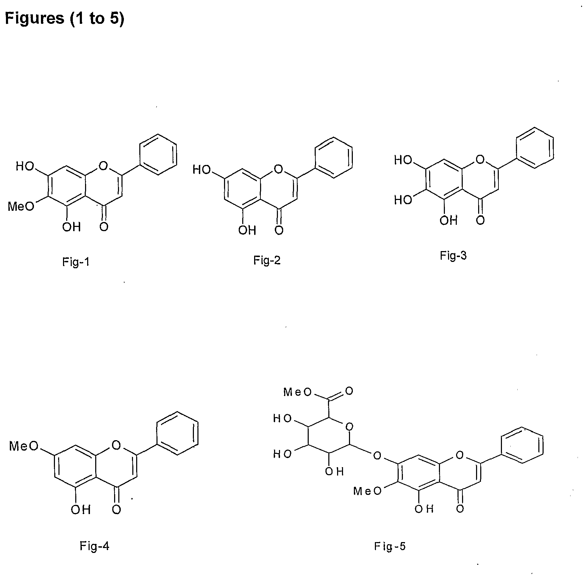

**Claims**

1. α-glucosidase inhibitory active fraction of plant *Oroxylum indicum*, selected from hexane and acetone extract of plant oroxylum indicum, wherein said hexane extract comprises oroxylin A, chrysin and baicalein and wherein said acetone extract comprises oroxylin A, chrysin, baicalein, methoxy chrysin and oroxyloside methyl ester, for use in in the treatment of post prandial hyperglycemia, hyperglycemia, diabetes mellitus, obesity and disease conditions requiring hyperglycemic control.

2. α-glucosidase inhibitory active fraction for use as claimed in claim 1 wherein the hexane extract of plant *Oroxylum indicum* comprises active compounds of oroxylin A, chrysin and baicalein and unidentified compounds in a ratio of about 60:10:25:5.0 (wt%).

3. α-glucosidase inhibitory active fraction for use as claimed in claim 1 wherein the acetone extract of plant *Oroxylum indicum* comprises the active compounds of oroxylin A, chrysin, baicalein, methoxy chrysin, oroxyloside methyl ester and unidentified compounds in a ratio of about 6.7:8.3:50:16.7:13.3:5.0 (wt%).

4. α-glucosidase inhibitory active fraction for use as claimed in claim 1, wherein the in vitro α- glucosidase inhibitory activity IC$_{50}$ of the hexane or acetone extract of *Oroxylum indicum* is in the range of 84 to 1 25 μg/mL.

5. α-glucosidase inhibitory active fraction for use as claimed in claim 1, wherein the hexane extract of plant *Oroxylum indicum* exhibits in vitro α-glucosidase inhibitory activity IC$_{50}$ of about 85 μg/mL.

6. α-glucosidase inhibitory active fraction for use as claimed in claim 1, wherein the acetone extract of plant *Oroxylum indicum* exhibits in vitro α -glucosidase inhibitory activity IC$_{50}$ of about 124 μg/mL.

7. Use of α-glucosidase inhibitory active fraction of plant *Oroxylum indicum,* selected from hexane and acetone extract of plant *Oroxylum indicum,* wherein said hexane fraction comprises oroxylin A, chrysin and baicalein and wherein said acetone fraction comprises oroxylin A, chrysin, baicalain, methoxy chrysin and oroxyloside methyl ester for the manufacture of a medicament for treating post prandial hyperglycemia, hyperglycemia, diabetes mellitus, obesity and disease condition requiring hyperglycemic control.

**Patentansprüche**

1. α-Glucosidase-hemmende aktive Fraktion der Pflanze *Oroxylum indicum,* ausgewählt aus dem Hexan- und Acetonextrakt der Pflanze *Oroxylum indicum,* wobei der Hexanextrakt Oroxylin A, Chrysin und Baicalein umfasst und wobei der Acetonextrakt Oroxylin A, Chrysin, Baicalein, Methoxychrysin und Oroxylosidmethylester umfasst, zur Verwendung bei der Behandlung von postprandialer Hyperglykämie, Hyperglykämie, Diabetes mellitus, Adipositas und von Krankheitszuständen, die eine hyperglykämische Kontrolle erfordern.

2. α-Glucosidase-hemmende aktive Fraktion zur Verwendung gemäß Anspruch 1, wobei der Hexanextrakt der Pflanze *Oroxylum indicum* die aktiven Verbindungen Oroxylin A, Chrysin und Baicalein und unidentifizierte Verbindungen in einem Verhältnis von etwa 60:10:25:5,0 (Gew.-%) umfasst.

**3.** α-Glucosidase-hemmende aktive Fraktion zur Verwendung gemäß Anspruch 1, wobei der Acetonextrakt der Pflanze *Oroxylum indicum* die aktiven Verbindungen Oroxylin A, Chrysin, Baicalein, Methoxychrysin, Oroxylosidmethylester und unidentifizierte Verbindungen in einem Verhältnis von etwa 6,7:8,3:50:16,7:13,3:5,0 (Gew.-%) umfasst.

**4.** α-Glucosidase-hemmende aktive Fraktion zur Verwendung gemäß Anspruch 1, wobei der IC$_{50}$-Wert der in-vitro-α-Glucosidase-hemmenden Aktivität des Hexan- oder Acetonextrakts von *Oroxylum indicum* im Bereich von 84 bis 125 μg/ml liegt.

**5.** α-Glucosidase-hemmende aktive Fraktion zur Verwendung gemäß Anspruch 1, wobei der Hexanextrakt der Pflanze *Oroxylum indicum* einen IC$_{50}$-Wert der invitro-α-Glucosidase-hemmenden Aktivität von etwa 85 μg/ml aufweist.

**6.** α-Glucosidase-hemmende aktive Fraktion zur Verwendung gemäß Anspruch 1, wobei der Acetonextrakt der Pflanze *Oroxylum indicum* einen IC$_{50}$-Wert der invitro-α-Glucosidase-hemmenden Aktivität von etwa 124 μg/ml aufweist.

**7.** Verwendung einer α-Glucosidase-hemmenden aktiven Fraktion der Pflanze *Oroxylum indicum,* die aus dem Hexan- und Acetonextrakt der Pflanze *Oroxylum indicum* ausgewählt ist, wobei der Hexanextrakt Oroxylin A, Chrysin und Baicalein umfasst und wobei der Acetonextrakt Oroxylin A, Chrysin, Baicalein, Methoxychrysin und Oroxylosidmethylester umfasst, zur Herstellung eines Medikaments zur Behandlung von postprandialer Hyperglykämie, Hyperglykämie, Diabetes mellitus, Adipositas und von Krankheitszuständen, die eine hyperglykämische Kontrolle erfordern.

## Revendications

**1.** Fraction active de la plante nommée *Oroxylum indicum* ayant une activité inhibitrice de l'α-glucosidase, choisie parmi des extraits à l'hexane et à l'acétone d'Oroxylum indicum, dans laquelle ledit extrait à l'hexane comprend de l'oroxyline A, de la chrysine et de la baicaléine et dans laquelle ledit extrait à l'acétone comprend de l'oroxyline A, de la chrysine, de la baicaléine, de la méthoxychrysine et de l'ester méthylique d'oroxyloside, destinée à être utilisée dans le traitement de l'hyperglycémie post-prandiale, de l'hyperglycémie, du diabète sucré, de l'obésité et des maladies nécessitant la régulation de l'hyperglycémie.

**2.** Fraction active inhibitrice de l'α-glucosidase destinée à être utilisée selon les termes de la revendication 1, dans laquelle l'extrait à l'hexane d'Oroxylum indicum comprend des composés actifs d'oroxyline A, de chrysine et de baicaléine et des composés non identifiés selon une proportion de 60:10:25:5,0 environ (% en poids).

**3.** Fraction active inhibitrice de l'α-glucosidase destinée à être utilisée comme revendiquée dans la revendication 1, dans laquelle l'extrait à l'acétone d'Oroxylum indicum comprend les composés actifs d'oroxyline A, de chrysine, de baicaléine, de méthoxychrysine et d'ester méthylique d'oroxyloside et des composés non identifiés selon une proportion de 6,7:8,3:50:16,7:13,3:5,0 environ (% en poids).

**4.** Fraction active inhibitrice de l'α-glucosidase destinée à être utilisée comme revendiquée dans la revendication 1, dans laquelle l'activité d'inhibition de l'α-glucosidase IC$_{50}$ de l'extrait à l'hexane ou à l'acétone d'*Oroxylum indicum* est comprise entre 84 et 125 μg/mL.

**5.** Fraction active inhibitrice de l'α-glucosidase destinée à être utilisée comme revendiquée dans la revendication 1, dans laquelle l'extrait à l'hexane d'*Oroxylum indicum* présente une activité d'inhibition de l'α-glucosidase in vitro IC$_{50}$ de 85 μg/mL environ.

**6.** Fraction active inhibitrice de l'α-glucosidase destinée à être utilisée comme revendiquée dans la revendication 1, dans laquelle l'extrait à l'acétone d'*Oroxylum indicum* présente une activité d'inhibition de l'α-glucosidase in vitro IC$_{50}$ de 124 μg/mL environ.

**7.** Utilisation de la fraction active de la plante nommée *Oroxylum indicum* ayant une activité inhibitrice de l'α-glucosidase, choisie parmi des extraits à l'hexane et à l'acétone d'*Oroxylum indicum,* dans laquelle ladite fraction extraite à l'hexane comprend de l'oroxyline A, de la chrysine et de la baicaléine et dans laquelle ladite fraction extraite à l'acétone comprend de l'oroxyline A, de la chrysine, de la baicaléine, de la méthoxychrysine et de l'ester méthylique d'oroxyloside pour la fabrication d'un médicament destinée à traiter l'hyperglycémie post-prandiale, l'hyperglycémie, le diabète sucré, l'obésité et les maladies nécessitant la régulation de l'hyperglycémie.

# Figures (1 to 5)

Fig-1

Fig-2

Fig-3

Fig-4

Fig-5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004122054 A, Rosner Harvey

**Non-patent literature cited in the description**

- **MOORARDIAN AD ; THURMAN JE.** *Drugs,* 1999, vol. 57, 19-29
- **UCHIDA, R ; NASU, A.** *Chem.. Pharm. Bull,* 1999, vol. 47, 187-193
- **KAUSIK, P ; DHAMAN A. K.** *The medicinal plants and crude drugs of India,* 2000, 398